# EUROPÄISCHE PATENTSCHRIFT

(11) **EP 1 594 613 B1**
(45) Veröffentlichungstag und Bekanntmachung des Hinweises auf die Patenterteilung: **17.05.2006**
(21) Anmeldenummer: 04710817.0
(22) Anmeldetag: 13.02.2004
(51) Int. Cl.: B01L 3/00, G01N 33/53

(54) **VERFAHREN ZUR UNTERSUCHUNG ZELLULÄRER PROBEN**
METHOD FOR THE INVESTIGATION OF CELLULAR SAMPLES
PROCEDE D'ANALYSE D'ECHANTILLONS CELLULAIRES

(30) Priorität: 13.02.2003 DE 20302263 U
(43) Veröffentlichungstag der Anmeldung: 16.11.2005
(73) Patentinhaber: Evotec AG, 22525 Hamburg (DE)
(72) Erfinder: SOLLBÖHMER, Olaf, D-21224 Rosengarten (DE); MICHAELSEN, Nico, D-22609 Hamburg (DE)
(74) Vertreter: von Kirschbaum, Alexander
(86) Internationale Anmeldenummer: PCT/EP2004/001357
(87) Internationale Veröffentlichungsnummer: WO 2004/071661

(56) Entgegenhaltungen:
- US-A- 6 037 171
- US-B1- 6 251 615
- US-B1- 6 368 838
- AMALDI U ET AL: "Cancer and radiation at the turn of the century" 0159-2101, USA SERIES: INTERNATIONAL CONGRESS SERIES (ISSN 0531-5131), 1994, Seiten 3-5, XP009033026 First International Symposium on Hadrontherapy;Como, Italy; October 18-21, 1993, Elsevier Science Publishers B.V., PO Box 211, Sara Burgerhartstraat 25, 1000 AE Amsterdam, Netherlands; Elsevier Science Publishing Co., Inc., P.O. Box 882, Madison Square Station, New York, New York 10159-2101, USA Ser ISBN: 0-444-81918-5
- PATENT ABSTRACTS OF JAPAN vol. 2000, no. 01, 31. Januar 2000 (2000-01-31) & JP 11 290062 A (NEC CORP), 26. Oktober 1999 (1999-10-26)

## Beschreibung

Die Erfindung betrifft ein Verfahren zur Untersuchung zellulärer Proben in einem Medium.

Bei Probenträgern handelt es sich beispielsweise um Titerplatten mit einer Vielzahl von Vertiefungen (Wells). Derartige Probenträger, die im Medium- und Hochdurchsatz-Screening eingesetzt werden, weisen beispielsweise 1.536 oder 2.080 Vertiefungen auf, die ein Volumen im Bereich von wenigen µl aufweisen. Da die sich in den Wells befindlichen Proben mit Hilfe von speziellen Mikroskopen beobachtet werden, sind die Vertiefungen bei Titerplatten, beispielsweise mit einem Bodenteil, verschlossen, wobei das Bodenteil transparent ist, insbesondere aus transparentem Glas besteht, so dass eine Beobachtung der Probe von unten möglich ist. Ebenso kann es sich bei dem Probenträger um einen Nano-Carrier oder Kultivierungstöpfe handeln. Ebenso kann es sich bei Probenträgern um ebene Probenträger, üblicherweise dünne Glasplättchen handeln. Des weiteren werden unter Probenträgern auch Träger, Platten oder dgl. mit Durchflusskanälen etc. verstanden, durch die ein Medium strömen kann.

Insbesondere bei der Untersuchung von Zellen besteht die Anforderung sowohl die Zelle selbst als auch den Überstand, d.h. die Umgebung der Zelle, zu beobachten. Der Überstand der Zelle wird beispielsweise zur Untersuchung von Stoffwechselprodukten beobachtet. Da die Lage der Zellen in dem Well jedoch undefiniert ist und der Überstand der Zellen nur in einem Bereich untersucht werden kann, in dem keine Zellen vorhanden sind, ist es zur Untersuchung des Überstandes erforderlich, derartige freie Flächen zu suchen, an denen sich keine Zellen befinden, um Untersuchungen, insbesondere mit Hilfe optischer Messmethoden, durch ein Bodenteil des Probenträgers hindurch durchzuführen. Dies muss durch einen Benutzer manuell erfolgen. Ebenso ist es möglich, zur Untersuchung des die Zellen umgebenden Mediums, d. h. des Überstandes mit einem zusätzlichen Objektiv eine Probenuntersuchung von oben durchzuführen. Dies ist beispielsweise möglich, wenn die Zellen am Wellboden haften. Die Untersuchung der Zellen selbst erfolgt sodann von unten durch den transparenten aus Kunststoff oder Glas bestehenden Boden. Die Untersuchung des Überstandes kann sodann von oben erfolgen. Hierzu ist jedoch ein äußerst aufwendiger Messaufbau erforderlich, da ein zusätzlicher Untersuchungspfad mit zusätzlichen optischen Einrichtungen und ggf. weiteren Detektoren vorgesehen sein muss.

Ein Probenträger, bei dem zur Untersuchung von Zellen einer biologischen Probe eine Vertiefung des Probenträgers beschichtet ist, um ein Anhaften der Zellen an dem Boden hervorzurufen, ist in US 6,037,171 beschrieben.

Aufgabe der Erfindung ist es, ein Verfahren zur Untersuchung zellulärer Proben in einem Medium zu schaffen, mit dem das die Zellen umgebende Medium auf einfache Weise untersucht werden kann.

Die Lösung der Aufgabe erfolgt erfindungsgemäß durch die Merkmale des Anspruchs 1.

Das erfindungsgemäße Verfahren zur Untersuchung zellulärer Proben in einem Medium erfolgt mit Hilfe eines Probenträgers, der insbesondere mehrere Vertiefungen aufweist, beispielsweise einer Titerplatte. Die folgende Beschreibung der Erfindung erfolgt beispielhaft anhand eines Probenträgers mit mehreren Vertiefungen (wells). Hierbei sind die Vertiefungen (wells) mit einem zur optischen Untersuchung der Probe transparentem Bodenteil verschlossen, wobei das Bodenteil vorzugsweise aus Glas besteht, jedoch auch als Kunststoffteil ausgebildet sein kann. An einer Innenseite mindestens einer der Vertiefungen ist partiell eine Beschichtung vorgesehen. Durch die Beschichtung wird das Anhaften von Zellen in dem beschichteten Bereich beeinflusst, d.h. insbesondere verbessert, verschlechtert oder vermieden. Dies führt dazu, dass sich an der Innenseite der Vertiefung mindestens eine Zellinsel bildet, wobei die Zellinsel jede beliebige Form aufweisen kann, insbesondere punkt- oder ringförmig ist. Die Zellinseln bilden sich insbesondere in einem Vorbereitungszeitraum, an den sich sodann ein Untersuchungszeitraum anschließen kann. Je nach Art des Mediums und der zellulären Probe kann ein sehr schnelles Bilden der Zellinseln erfolgen, so dass die Untersuchung bereits während des Vorbereitungszeitraums erfolgen kann bzw. eine genau Trennung zwischen Vorbereitungszeitraum und Untersuchungszeitraum nicht möglich bzw. nicht erforderlich ist. Nach diesem Zeitraum sind die Zellinseln sodann von Medium, bzw. dem Überstand der Zellen, umgeben. Es ist somit möglich, das Medium zu untersuchen, wobei auf Grund der Bildung von Zellinseln sichergestellt ist, dass die Untersuchung des Zellüberstandes nicht durch die Zellen selbst beeinflusst wird (z.B. in Suspension vorliegende Zellen oder im Sedimentationsvorgang befindliche Zellen). Auf Grund des Erzeugens von Zellinseln kann sichergestellt werden, dass die Untersuchung des Mediums definiert außerhalb der Zellen stattfindet. Es ist somit möglich, neben den Zeilinseln eine Untersuchung des die Zellinseln umgebenden Mediums ebenfalls von unten durchzuführen. Dies hat den Vorteil, dass mit Hilfe der selben Untersuchungsvorrichtung von unten durch den Zellboden einerseits die Zellen selbst und andererseits auch der Zellüberstand vorzugsweise mit Hilfe optischer Messmethoden untersucht werden kann. Ein komplizierter apparativer Aufbau, der zusätzlich eine Untersuchung der Probe von oben ermöglicht, ist somit nicht erforderlich. Da die Lage der Zellinseln auf Grund der partiellen Beschichtung der Innenseite der Vertiefung vordefiniert werden kann und sodann bekannt ist, ist ein manuelles Aufsuchen freier Flächen nicht erforderlich. Dies hat den erheblichen Vorteil, dass es mit Hilfe des erfindungsgemäßen Verfahrens möglich ist, Zellüberstände im Screeningverfahren, insbesondere im High-Throughput und Medium-Throughput Screening durchzuführen. Es ist somit ein hoher Grad an Automatisierung der Untersuchung von Zellüberständen möglich.

An Stelle eines, vorzugsweise einer Titerplatte mit mehreren Vertiefungen, (wells) ausgebildeten Probenträgers kann auch ein beispielsweise ebener Probenträger vorgesehen sein. Insbesondere handelt es sich bei dem ebenen Probenträger um ein aus Kunststoff oder Glas bestehendes Plättchen, das sodann dem Bodenteil entspricht. Auf einer Innenseite, d. h. auf einer Oberseite des Bodenteils bzw. Plättchens sind sodann die partiellen Beschichtungen vorgesehen. Die zelluläre Probe kann sodann beispielsweise in Form von Tröpfchen auf die entsprechenden Bereiche aufgebracht werden. Hierbei ist die Beschichtung teilweise oder vollständig innerhalb des Tröpfchens angeordnet. Die Größe des Tröpfchens bzw. der Beschichtung ist hierbei vorzugsweise derart gewählt, dass ein Teil des Tröpfchens stets außerhalb der Beschichtung angeordnet ist, so dass im Überstand der Zellinsel Untersuchungen durchgeführt werden können. Es ist somit möglich, durch Beschichtung der Probenträger wie z.B. einer planaren Platte (z.B. aus Kunststoff oder Glas) mittels des erfindungsgemäßen Verfahrens Zellsuspensionen direkt auf diese aufzutropfen, die Zellen sedimentieren zu lassen und eine Messung im flüssigen Medium derartiger Tropfen durchzuführen.

Weitere geeignete Probenträger weisen insbesondere Mikrokanalstrukturen auf, wie z.B. Durchflusskanalstrukturen, durch die eine Zellsuspension geleitet werden kann, so dass die Zellen sodann an den beschichteten Stellen anhaften bzw. von diesen ferngehalten werden. Bei den Mikrokanalstrukturen kann es sich vorzugsweise um ein System mehrerer Kanäle handeln, die verzweigt vorliegen. So kann z.B. in einem Hauptkanal eine für einen Zelltyp 1 spezifische Adhäsionsbeschichtung vorliegen. Bei Durchleiten einer Zellsuspension, welche neben Zellen des Zelltyps 1 auch andere Zelltypen enthält, verbleiben die Zelltyp-1-Zellen im Hauptkanal, während die anderen Zellen z.B. in einem Separationskanal abgeleitet werden. Auf diese Weise kann vor der Untersuchung der Zellen des ersten Zelltyps in vorteilhafter Weise eine Separation vorgenommen werden.

Der Begriff "zelluläre Proben" umfasst neben eigentlichen Zellen auch andere biologische Partikel, insbesondere zelluläre Membranvesikel oder virusähnliche Partikel, die beispielsweise zelluläre Rezeptoren an ihrer Oberfläche aufweisen. Dementsprechend ist der Begriff "Zellinsel" analog zu verstehen. An Stelle von zellulären Proben ist das erfindungsgemäße Verfahren somit beispielsweise auch mit Proben durchführbar, die Partikel aufweisen, wobei die Partikel sodann an den beschichteten Bereichen Partikelinseln bilden.

Mit Hilfe des erfindungsgemäßen Verfahrens können insbesondere Sekretionsassays durchgeführt werden. Hierbei handelt es sich um die Durchführung von Untersuchungen, in denen die Sekretionsprodukte, die von den Zellen in das Medium abgegeben werden, gemessen werden. Dies kann ebenfalls wieder im Screeningverfahren bei einer Vielzahl von Proben erfolgen, die beispielsweise in einer Microtiterplatte angeordnet sind. Die Messung erfolgt hierbei vorzugsweise über optische Messverfahren, wie Fluoreszenzmessverfahren, wobei das Sekretionsprodukt beispielsweise selbst fluoresziert. Ebenso kann ein Fusionsprotein mit einem fluoreszierenden Protein (beispielsweise GFP) exprimiert werden. Anschließend kann sodann die sekretierte Verbindung im Überstand der Zellen von unten, d.h. durch das Bodenteil hindurch, gemessen werden.

Ferner ist es möglich, zu der Detektion von Sekretionsprodukten die Lumineszenz , insbesondere die Fluoreszenz einer mit dem Sekretionsprodukt interagierenden Substanz zu messen. Bei der interagierenden Substanz kann es sich beispielsweise um ein synthetisches Bead handeln, das mit einem entsprechenden Marker markiert ist. Hierbei wird vorzugsweise die Änderung der Lumineszenz bzw. Fluoreszenz nach der Bindung an ein Sekretionsprodukt detektiert. Ferner können als interagierende Substanzen auch markierte Antikörper oder sonstige biologische/ chemische Sonden verwendet werden, die selektiv mit dem Sekretionsprodukt reagieren. Auch hierbei kann die Änderung der Lumineszenz bzw. Fluoreszenz Aufschluss über das Sekretionsprodukt liefern.

Bei einem weiteren bevorzugten Verfahren wird die Aufnahme von Stoffen aus dem Medium bzw. dem Überstand durch die Zellen gemessen. Auch hierbei erfolgt die Messung vorzugsweise über Lumineszenz- bzw. Fluoreszenzsignale. Das Signal ändert sich hierbei bei der Aufnahme einer Substanz in die Zelle. Dies ist beispielsweise bei der Untersuchung der Funktion von Ionenkanälen möglich, bei der der Transport von Farbstoffen durch die Zellmembran untersucht werden kann.

Vorzugsweise ist das Bodenteil, um gute Messergebnisse erzielen zu können, aus optisch hochwertigem Glas hergestellt, das vorzugsweise eine Dicke von 100 - 1.000 µm, insbesondere 100 - 300 µm, aufweist.

Die Messung innerhalb des Überstandes erfolgt vorzugsweise durch konfokale Spektroskopie oder konfokale bildgebende Verfahren. Es ist auch möglich andere Fluoreszenzreader zu verwenden, die ein begrenztes Messvolumen in Z-Richtung, d. h. senkrecht zum Bodenteil, aufweisen. In dem Anwendungsbereich sind auch konventionelle Reader einsetzbar, mit denen keine konfokale Messung durchgeführt wird. Die Verwendung von konfokalen Readern ist jedoch insofern vorteilhaft, als dass das Messvolumen insbesondere in Z-Richtung wohl definiert ist. Somit lässt sich gezielt mit einem apparativen Aufbau sowohl eine Messung in bzw. an den Zellen als auch eine Untersuchung des Mediums durchführen.

Ein besonderer Vorteil des erfindungsgemäßen Verfahrens besteht darin, dass die Zwischenräumen zwischen den Zellinseln nicht exakt bestimmt werden müssen, sondern bereits durch die Lage der Beschichtungen definiert sind. Somit lassen sich vorzugsweise automatisiert Zwischenräume zwischen derartigen Inseln gezielt steuern. Es ist ferner ausreichend, mit einem insbesondere eine hohe Apertur aufweisendem Objektiv bezogen auf das Bodenteil des Probenträgers tief in den Überstand hinein zu fokussieren. Hierdurch werden die Zellen bei herkömmlicher Abbildung unscharf und bei konfokaler Abbildung gar nicht abgebildet. Die hierbei ggf. entstehenden Störsignale können eliminiert werden. Dies hat eine geringe Signalabschwächung zur Folge, die je nach Art der Untersuchung akzeptabel ist.

Der Probenträger zur Durchführung des vorstehenden Verfahrens weist an einer Innenseite mindestens eine Vertiefung oder eine Kanalstruktur mit einer partiellen Beschichtung an einer Innenseite der Vertiefung/ der Kanalstruktur auf. Hierbei handelt es sich um eine Beschichtung, die das Anhaftverhalten der Partikel in dem beschichteten Bereich beeinflusst, d.h. insbesondere verbessert oder verschlechtert bzw. verhindert. Die anhaftenden Partikel, bei denen es sich insbesondere um Zellen, vorzugsweise um adhärente Zelleri, handelt, haften somit an den Stellen mit verbessertem Anhafteverhalten des Wells. Es findet somit innerhalb des Wells einer Kanalstruktur eine Konzentration von Zellen in bestimmten Bereichen statt. Ferner bilden sich an den Stellen mit verschlechterter Anhaftung "Fenster" aus, an denen keine oder nur eine äußerst geringe Anzahl an Zellen anwachsen. Diese Bereiche können zur Untersuchung von Stoffwechselprozessen u.dgl., d.h. für Untersuchungen im Überstand der Zelle, genutzt werden. Da diese Bereiche eine vordefinierte Größe und ggf. auch eine vordefinierte Lage haben, sind sie auf einfache Weise auffindbar. Insbesondere ist es möglich, diese Bereiche vollautomatisch zu untersuchen. Wenn die Lage der Fenster bzw. der beschichteten Bereiche bekannt ist, ist es auf einfache Weise möglich, den Probenträger oder die Untersuchungsoptik entsprechend gegeneinander zu verfahren. Selbst wenn die Lage der Beschichtung nicht exakt bekannt ist, ist es durch das Vorsehen von Beschichtungen möglich, mit Hilfe einer Bildverarbeitungssoftware die entsprechenden Bereiche in dem Well zu detektieren. Dies ist insbesondere möglich, da die beschichteten Bereiche vorzugsweise eine Mindestgröße aufweisen. Die Größe der beschichteten Bereiche ist vorzugsweise größer als 0,01 mm².

Wie vorstehend insbesondere anhand eines Probenträgers, wie einer Titerplatte, beschrieben, sind als Probenträger auch die bereits anhand des Verfahrens beschriebenen unterschiedlichen Probenträger einsetzbar.

Insbesondere ist es mit Hilfe eines Probenträgers möglich, Proben, die Partikel, insbesondere Zellen, enthalten, mit einem Imagesystem, beispielsweise mit konfokaler Optik zu analysieren. Bei einer Zellen enthaltenden Probe kann die Analyse der Zellen sowie des Überstandes zeitlich nacheinander, beispielsweise durch zeitaufgelöste Fluoreszenzspektroskopie, wie Fluoreszenzkorrelationsspektroskopie (FCS) oder auch andere bekannte fluoreszenzspektroskopische Methoden, wie Fluoreszenzintensitätsverteilungsverfahren (FIDA, 2D-FIDA), Fluoreszenzlebenszeitmessungen oder Polarisationsmessungen, oder auch simultan erfolgen. Beispielsweise kann der Überstand mit einer zweiten Detektionsmethode, beispielsweise FCS, untersucht werden.

Besonders bevorzugt ist es, die Beschichtung an einer Bodenfläche des Wells vorzusehen. Die Beschichtung ist somit innerhalb des Wells an dessen Boden angeordnet.

Vorzugsweise sind an der Innenseite, besonders bevorzugt an der Bodenfläche der Wells mehrere beschichtete und unbeschichtete Bereiche vorgesehen. Diese können unregelmäßig oder regelmäßig angeordnet sein.

Zum Erzeugen der Beschichtung ist es besonders bevorzugt, ein Beschichtungsmedium in das Well zu tropfen. Das Tropfen kann mittels einer Pipette oder einem Dispenser erfolgen. Dies hat den Vorteil, dass äußerst kleine Tropfen abgegeben werden können, die sodann nur einen Teil der Bodenfläche bedecken.

Als Beschichtungsmedium kann beispielsweise Collagen, Fibronektin, Laminin, Poly-D-Lysin verwendet werden.

Anstatt in einer Vertiefung mehrere Bereiche vorzusehen, die beschichtet oder unbeschichtet sind, ist es auch möglich, mehrere, vorzugsweise zwei Bereiche vorzusehen, die unterschiedliche Beschichtungen aufweisen. Hierbei wird durch eine Beschichtung beispielsweise das Anhaften von Zellen gefördert und durch die andere Beschichtung verhindert bzw. verschlechtert.

Nachfolgend wird die Erfindung anhand einer bevorzugten Ausführungsform unter Bezugnahme auf die anliegenden Zeichnungen näher erläutert.

Es zeigen:
- Fig. 1: eine schematische Draufsicht einer Titerplatte,
- Fig. 2: eine schematische Schnittansicht entlang der Linie II-II,
- Fig. 3: eine schematische vergrößerte Darstellung einer Draufsicht einer Vertiefung und

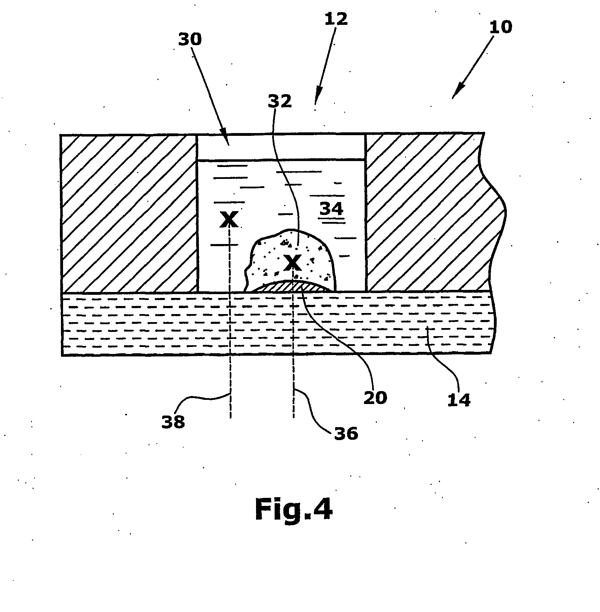

- Fig.4: eine schematische vergrößerte Schnittansicht einer Vertiefung mit einer zellulären Probe.

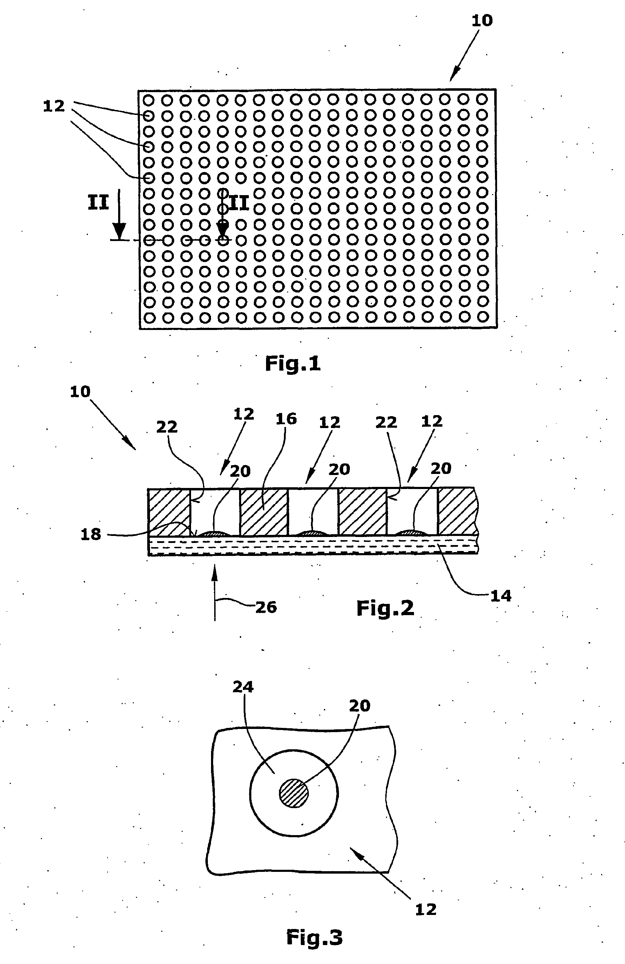

Ein Probenträger 10, wie eine Titerplatte, weist mehrere in Zeilen und Spalten angeordnete Vertiefungen bzw. Wells 12 auf. Die Wells 12 sind im dargestellten Ausführungsbeispiel dadurch hergestellt, dass der Probenträger 10 durchgehende Öffnungen aufweist, die mit einem Bodenteil 14 verschlossen sind. Das Bodenteil 14 ist transparent und vorzugsweise aus Glas. Ferner ist es möglich, einen die durchgehenden Bohrungen aufweisenden Grundträger 16 (Fig. 2) und das Bodenteil 14 einteilig auszubilden. An einer Bodenfläche 18 ist eine Beschichtung 20 angeordnet. Die Beschichtung 20 kann auch anstatt an einer Bodenfläche 18 an einer anderen Innenseite des Wells, wie beispielsweise der Innenwand 22, vorgesehen sein.

Im dargestellten Ausführungsbeispiel ist mit Hilfe einer Pipette oder einem Dispenser ein Tropfen Beschichtungsmedium in das Well 12 getropft worden, so dass sich an der Bodenfläche 18 eine im Wesentlichen kreisrunde Beschichtung 20 ausbildet. Auf Grund der Viskosität des Beschichtungsmediums ist der beschichtete Bereich 20 leicht gewölbt (Fig. 2). Der beschichtete Bereich 20 ist von einem nicht beschichteten Bereich 24 (Fig. 3) umgeben. Das Beschichtungsmittel wird insbesondere als wässrige Lösung aufgefangen und trocknet ein (oder an).

In das Well 12 wird zur Untersuchung einer Probe diese in Fig. 2 von oben eingefüllt. Die beispielsweise in der Probe vorhandenen Zellen wachsen beispielsweise im Bereich der Beschichtung 20 an die Bodenfläche 18 an. Im Bereich 24, der keine Beschichtung oder eine Beschichtung aus einem anderen, das Anwachsen verschlechternden Medium aufweist, wachsen keine oder zumindest eine erheblich geringere Anzahl an Zellen an. Die Beobachtung des Wells 12. erfolgt durch eine geeignete Optikeinrichtung in Fig. 2 von unten in Richtung eines Pfeils 26 durch das Bodenteil 14 hindurch.

In dem dargestellten Well 12 ist eine flüssige zelluläre Probe 30 vorgesehen. Nach einem von der Art der Probe abhängigen Zeitraum bildet sich auf der Beschichtung 20 eine Zellinsel 32, die von Medium bzw. Überstand 34 umgeben ist. Über eine unterhalb des Probenträgers 10 angeordnete, nicht dargestellte Untersuchungseinrichtung ist es möglich, entlang eines gestrichelt dargestellten Strahlengangs 36 innerhalb der Zellinsel 32 angeordnete Zellen zu untersuchen. Durch ein leichtes Verschieben des Probenträgers 10 und/ oder der Untersuchungsvorrichtung ist es femer möglich, entlang eines Untersuchungsstrahlengangs 38 im Überstand 34 Untersuchungen durch zuführen. Es ist somit auf einfache Weise möglich, im Überstand 34 Sekretionsprodukte, insbesondere mit Hilfe optischer Messverfahren zu ermitteln. Ebenso ist es möglich, die Aufnahme von Stoffen durch die Zellen aus dem Überstand 34 zu untersuchen.

## Patentansprüche

1. Verfahren zur Untersuchung zellulärer Proben in einem Medium, die in/ auf einem Probenträger (10) angeordnet sind, wobei der Probenträger ein zur optischen Untersuchung der Probe transparentes Bodenteil (14) aufweist und an einer Innenseite (18, 22) mindestens partiell eine Beschichtung (20) vorgesehen ist, wobei durch die Beschichtung ein Anhaftverhalten der in der Probe vorhandenen zellulären Partikel in dem beschichteten Bereich (20) beeinflusst ist, so dass sich Zellinseln (32) bilden und das die Zellinseln (32) umgebende Medium (34) untersucht wird.

2. Verfahren zur Untersuchung zellulärer Proben nach Anspruch 1, bei welchem die Untersuchung des Mediums (34) mit optischen Messmethoden durch das Bodenteil (14), in Bereichen erfolgt, die frei von Zellinseln sind.

3. Verfahren zur Untersuchung zellulärer Proben nach Anspruch 1 oder 2, bei welchem Sekretionsprodukte der Zellen in dem Medium (34) gemessen werden.

4. Verfahren zur Untersuchung zellulärer Proben nach Anspruch 1 oder 2, bei welchem die Aufnahme von Stoffen aus dem Medium (34) durch die Zellen gemessen wird.

5. Verfahren nach einem der Ansprüche 1 - 4, **dadurch gekennzeichnet, dass** die Beschichtung durch Auftropfen von Beschichtungsmedium erfolgt.

## Claims

1. A method for examining cellular samples in a medium, said cellular samples being arranged in/on a sample carrier (10), wherein said sample carrier comprises a transparent bottom portion (14) allowing optical examination of the sample, and at least partially comprises a coating (20) on the inside (18,22), wherein said coating influences, in the coated region (20), the adhesion behavior of the cellular particles included in the sample such that cell islands (32) are formed, and the medium (34) surrounding said cell islands (32) is examined.

2. The method for examining cellular samples according to claim 1, wherein examination of the medium (34) is carried out with the aid of optical measuring methods through the bottom portion (14) in regions which are free from cell islands.

3. The method for examining cellular samples according to claim 1 or 2, wherein secretion products of the cells are measured in the medium (34).

4. The method for examining cellular samples according to claim 1 or 2, wherein absorption of substances from the medium (34) by the cells is measured.

5. The method according to one of claims 1-4, **characterized in that** coating is effected by dropping of coating medium.

## Revendications

1. Procédé d'analyse d'échantillons cellulaires dans un milieu, qui sont disposés dans/sur un porte-échantillon (10), le porte-échantillon comportant un élément de fond (14) transparent pour permettre un examen optique de l'échantillon, et un revêtement (20) étant prévu au moins partiellement sur une face intérieure (18, 22), le revêtement influençant le comportement d'adhérence des particules cellulaires présentes dans l'échantillon dans la zone revêtue (20) de telle sorte qu'il se forme des îlots cellulaires (32), et que l'on analyse le milieu (34) entourant les îlots cellulaires (32).

2. Procédé d'analyse d'échantillons cellulaires selon la revendication 1, dans lequel l'analyse du milieu (34) est réalisée par des méthodes optiques de mesure à travers la partie de fond (14), dans des zones exemptes d'îlots cellulaires.

3. Procédé d'analyse d'échantillons cellulaires selon la revendication 1 ou 2, dans lequel on mesure des produits de sécrétion des cellules dans le milieu (34).

4. Procédé d'analyse d'échantillons cellulaires selon la revendication 1 ou 2, dans lequel on mesure l'absorption, par les cellules, de substances provenant du milieu (34).

5. Procédé selon l'une des revendications 1 à 4, **caractérisé en ce que** le revêtement est réalisé par application de gouttes du milieu de revêtement.
